**Europäisches Patentamt**

**European Patent Office**

**Office européen des brevets**

(19)

(11) Publication number : **0 377 037 B1**

# EUROPEAN PATENT SPECIFICATION

(12)

(45) Date of publication of patent specification :
**07.06.95 Bulletin 95/23**

(21) Application number : **88906090.1**

(22) Date of filing : **14.07.88**

(86) International application number :
**PCT/JP88/00701**

(87) International publication number :
**WO 89/00691 26.01.89 Gazette 89/03**

(51) Int. Cl.⁶ : **G01N 27/30**

(54) ENZYME SENSOR AND METHOD OF MANUFACTURING SAME.

(30) Priority : **16.07.87 JP 176012/87**

(43) Date of publication of application :
**11.07.90 Bulletin 90/28**

(45) Publication of the grant of the patent :
**07.06.95 Bulletin 95/23**

(84) Designated Contracting States :
**DE FR**

(56) References cited :
**JP-A- 6 232 351**
**JP-A- 6 275 346**
**JP-A-62 235 557**
**JP-A-62 240 849**

(73) Proprietor : **TERUMO KABUSHIKI KAISHA**
**No. 44-1, Hatagaya 2-chome,**
**Shibuya-ku**
**Tokyo 151 (JP)**

(72) Inventor : **KATSUBE, Teruaki**
**1-1-205, Nagayama 3-chome**
**Tama-shi Tokyo 206 (JP)**

(74) Representative : **Gillard, Marie-Louise et al**
**Cabinet Beau de Loménie**
**158, rue de l'Université**
**F-75340 Paris Cédex 07 (FR)**

## Description

Technical Field

The present invention relates to an enzyme sensor and, more particularly, to a small and simple enzyme sensor which has a simple arrangement and high measurement precision and can convert a voltage generated in accordance with an enzyme concentration into a current value by a low-impedance circuit to measure the voltage and a method of manufacturing the same.

Background Art

An FET has been conventionally used as an enzyme sensor because the FET has a good response characteristic. An FET enzyme sensor has been fabricated by coating an enzyme layer or a combination of an enzyme layer and another layer on a gate isolating membrane.

The FET enzyme sensor of this type, however, is still unsatisfactory in reliability and stability and also poses a problem of drift and the like.

It has also been described in JP-A-62-32351 enzyme sensors which include hydrogen peroxide electrodes.

Disclosure of Invention

It is an object of the present invention to provide a small and simple enzyme sensor which has a simple arrangement and high measurement precision and a method of manufacturing the same.

In order to achieve the above object, an enzyme sensor of the present invention comprises: an enzyme-sensitive portion; a reference electrode portion; and a detecting means for receiving a potential difference generated across the enzyme-sensitive portion and the reference electrode portion with a load of a low impedance, for converting the potential difference into a current, and for detecting the current. Characteristically the load of a low impedance is 100Ω-10MΩ.

The enzyme-sensitive portion preferably comprises an electrically conductive layer coated on an isolating substrate, and an enzyme fixed layer covering the electrically conductive layer and holding an enzyme.

The electrically conductive layer is an iridium oxide layer.

The enzyme fixed layer carries urease.

The reference electrode portion is located near the enzyme-sensitive portion on the isolating substrate and comprises an iridium oxide layer and a layer covering the iridium oxide layer and not holding the enzyme.

A method of manufacturing an enzyme sensor according to the present invention comprises the steps of: forming a plurality of electrically conductive layers separated from each other on an isolating substrate; coating an enzyme fixed layer holding an enzyme on a part of the electrically conductive layer; coating a layer not holding the enzyme on the remaining part of the electrically conductive layer; and connecting at an end not dipped in a solution to be examined, the electrically conductive layer covered with the enzyme fixed layer and the electrically conductive layer covered with the layer not holding the enzyme with detecting means for receiving a potential difference generated across the two electrically conductive layers with a load of a low impedance, for converting the potential difference into a current, and for detecting the current. Characteristically the load of a low impedance is 100Ω-10MΩ.

According to the present invention, there is provided a small and simple enzyme sensor which has a simple arrangement and high measurement precision.

Brief Description of Drawings

Fig. 1A is a plan view showing an arrangement of an enzyme sensor according to an example of the present invention;

Fig. 1B is a schematic view showing a part of an arrangement of an enzyme sensor according to another example of the present invention;

Fig. 2A is a graph showing a reaction obtained when a resistance of the enzyme sensor of the present invention is set to be 1 kΩ;

Fig. 2B is a graph showing a reaction obtained when the resistance of the enzyme sensor of the present invention is set to be 10 kΩ; and

Fig. 2C is a graph showing a reaction obtained when the resistance of the enzyme sensor of the present invention is set to be 1 MΩ.

Best Mode of Carrying Out the Invention

The present invention will be described in detail below by way of its examples with reference to the accompanying drawings.

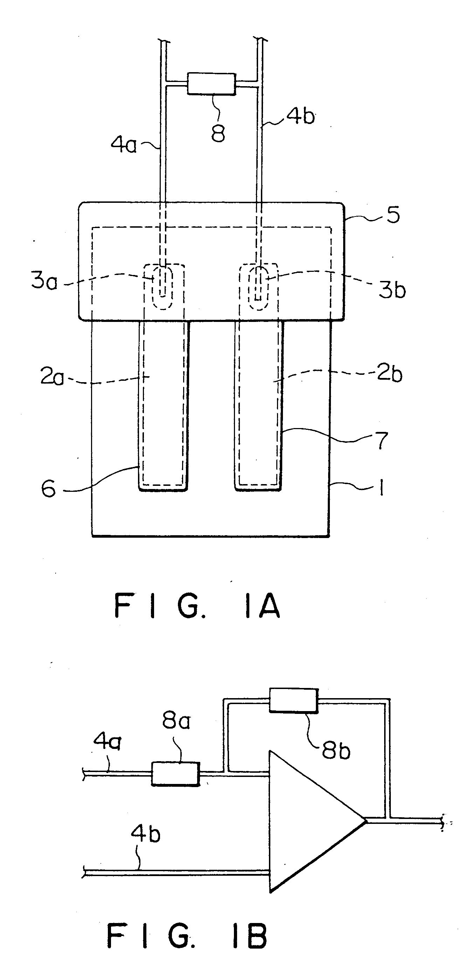

Fig. 1 is a plan view showing an arrangement of an enzyme sensor according an example of the present invention.

Referring to Fig. 1, the enzyme sensor of the present invention and a method of manufacturing the same will be described below. In this example, a voltage generated in accordance with an enzyme concentration is extracted as a current value by a low-impedance circuit and then converted into a voltage again to be measured.

(1) Formation of iridium oxide (IrOx) layer

In this example, glass 1 (slide glass normally used in a microscope and the like) was used as an isolating substrate. Two iridium oxide layers 2a and 2b (layer thickness = 800 Å, width = 1.8 mm and length = 5 mm) were formed on the surface of the glass 1 by sputtering. Lead wires 4a and 4b were connected to the iridium oxide layers 2a and 2b by electrically conductive adhesives 3a and 3b, respectively. A connection portion was isolated by an isolating adhesive 5. The thickness of each of the iridium oxide layers 2a and 2b is preferably 200 to 5,000 Å, more preferably, 500 to 1,500 Å and, most preferably, 600 to 1,000 Å. If the layer is thin, a layer resistance is undesirably increased to render a low impedance meaningless. On the contrary, if the layer is thick, a problem of removal of the layer arises.

As the isolating substrate, sapphire, ITO (iridium tin oxide), glass or quartz may be used. The iridium oxide layers 2a and 2b consist of an electrically conductive substance capable of generating a voltage between this substance and an enzyme fixed layer to be described next in accordance with an enzyme concentration.

(2) Formation of enzyme fixed layer

An enzyme fixed layer 6 holding urease was coated on the iridium oxide layer 2a formed in the step of item (1).

More specifically, 5 μl of a solution prepared by dissolving urease (manufactured by TOYOBO CO., LTD) in a 0.2-M tris-hydrochloric acid buffer solution (pH = 8.5) - a 15% bovine serum albumin solution (manufactured by Hani Chemicals K.K.) were extracted by a microsyringe and then coated and air-dried on an exposed surface of the iridium oxide. Thereafter, 0.5 μl of a 25-vol% glutaraldehyde solution were dropped on the resultant material, thereby forming the urease-holding enzyme fixed layer 6.

An albumin layer 7 not holding urease was coated on the other iridium oxide layer 2b.

(3) Setting of input impedance

While resistors 8 having three different resistances shown in Table 1 below were sequentially connected to the lead wires 4a and 4b, urea was dropped, and changes in generated potential differences were measured.

Table 1

| Resistance | 1 kΩ | 10 kΩ | 1 MΩ |
|---|---|---|---|

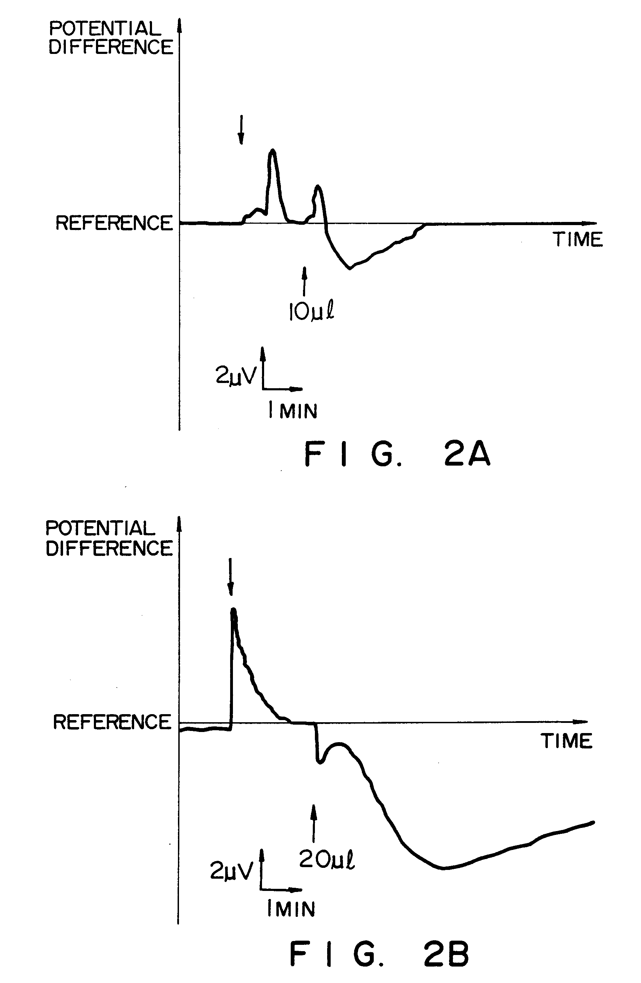

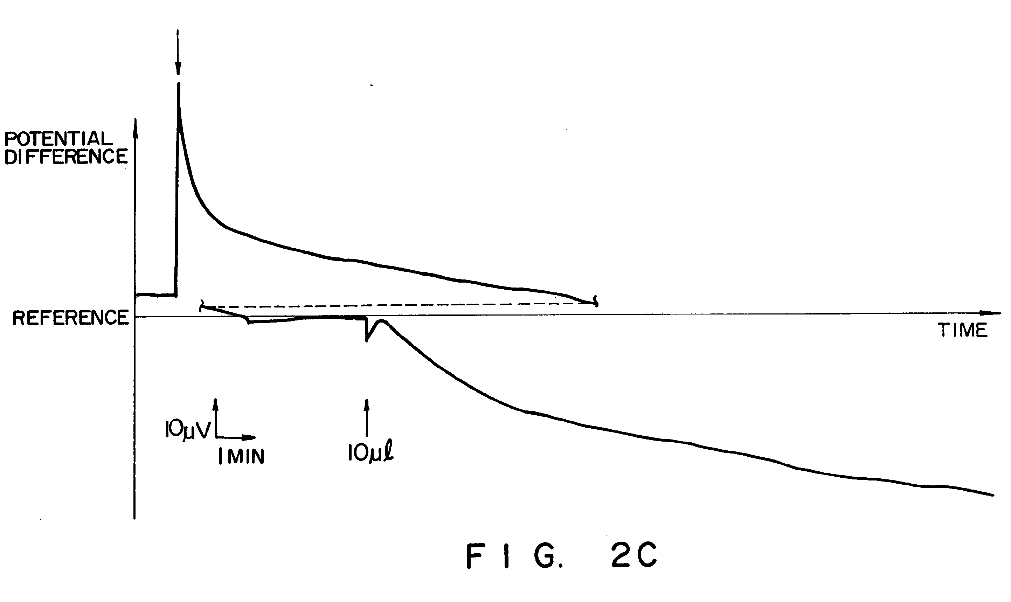

The measurement results are shown in Figs. 2A, 2B and 2C. In Figs. 2A to 2C, each white arrow represents a drop timing of a phosphoric acid buffer solution, and each black arrow represents a drop timing of a urea solution. Each value represents an injection amount of a 2-mg/cc urea solution injected in 20 μl of the phosphoric acid solution. Fig. 2A corresponds to the resistance of 1 kΩ; Fig. 2B, that of 10 kΩ; and Fig. 2C, that of 1 MΩ. If the resistance is too high, a response characteristic is degraded; if it is too small, a drift is increased. Therefore, the resistance is preferably 100Ω to 10 MΩ and, more preferably, 1 kΩ to 1 MΩ.

In Figs. 2A to 2C, a urea concentration is measured as a value changed by dropping of the urea solution from a stable state (flat portion) after the phosphoric acid buffer solution is dropped. For this reason, in Figs. 2A to 2C, the stable state obtained after dropping of the phosphoric acid buffer solution is used as a basis of the measurement. A stable state before dropping of the phosphoric acid buffer solution is a dry state and substantially equal to the reference value. The measurement, however, can be performed more accurately on the basis of the stable state after dropping of the phosphoric acid buffer solution. As is apparent from Figs. 2A to

3

2C, a drift is smallest and the stability is best when the resistance is 10 kΩ. That is, the resistance of 10 kΩ is best suited to urea concentration measurement in terms of a response speed to dropping of the urea solution.

As has been described above, by decreasing the input impedance to a predetermined value (100Ω to 10 MΩ), there is provided an enzyme sensor with higher reliability and stability and a smaller drift than those of a conventional enzyme sensor which performs measurement using an FET or the like having a high input impedance.

In addition, the enzyme sensor having a low input impedance according to the present invention has the following features.

(1) The sensor is very stable to a noise drift.

(2) The sensor requires only a very small amount of a solution to be examined.

(3) The sensor is simple in arrangement.

As shown in Fig. 1B, resistors 8a and 8b in place of the resistor 8 in Fig. 1A may be connected to the lead wires 4a and 4b. In this case, a current flowing through the resistor 8b is measured, i.e., a voltage generated in accordance with an enzyme concentration is measured as a current value to achieve the same results. In addition, another circuit arrangement capable of decreasing an input impedance can be similarly used. That is, a circuit arrangement is not limited to those of the above examples.

In each of the above examples, the enzyme sensor has the simplest arrangement. The enzyme sensor, however, may be of a composite type obtained by forming a plurality of iridium oxide layers. Furthermore, in addition to iridium oxide, palladium oxide or ruthenium oxide may be used. Also, in each of the above examples, the urea sensor has been described as a typical example of an enzyme sensor. The present invention, however, can be applied to another enzyme sensor.

Moreover, a method of forming an iridium oxide or enzyme fixed layer is not limited to that of the above examples.

It is to be understood that the present invention includes all the changes and additions that fall within the scope of the appended claims.

## Claims

1. An enzyme sensor comprising:

an enzyme-sensitive portion (2a, 6);

a reference electrode portion (2b, 7); and

detecting means for receiving a potential difference generated across said enzyme-sensitive portion (2a, 6) and said reference electrode portion (2b, 7), for converting the potential difference into a current and for detecting said current;

wherein said potential difference is received with a load (8,8a, 8b) of 100Ω-10MΩ impedance.

2. A sensor according to claim 1, wherein said enzyme-sensitive portion (2a, 6) comprises:

- an electrically conductive layer (2a) coated on an isolating substrate (1); and
- an enzyme fixed layer (6) covering said electrically conductive layer (2a) and holding an enzyme.

3. A sensor according to claim 2, wherein said electrically conductive layer (2a) is an iridium oxide layer.

4. A sensor according to claim 2 or 3, wherein said enzyme fixed layer (6) carries urease.

5. A sensor according to anyone of claims 2 to 4 wherein said reference electrode portion (2b, 7) is located near said enzyme fixed layer (6) on said isolating substrate (1) and comprises:

- an iridium oxide layer (2b) on said isolating substrate (1)
- an enzyme-free layer (7) covering said iridium oxide layer (2b).

6. A method of manufacturing an enzyme sensor according to anyone of claims 1 to 5, comprising the steps of

- forming a plurality of electrically conductive layers (2a, 2b) separated from each other on an isolating substrate (1);
- coating an enzyme fixed layer (6) holding an enzyme on part (2a) of said electrically conductive layers;
- coating an enzyme-free layer (7) on the remaining part (2b) of said electrically conductive layers;
- connecting, at an end not dipped in the solution to be examined, said electrically conductive layer

4

(2a) covered with said enzyme fixed layer (6) and said electrically conductive layer (2b) covered with said enzyme-free layer (7) with detecting means for receiving a potential difference generated across said two electrically conductive layers (2a, 2b), for converting the potential difference into a current and for detecting said current;

wherein said potential difference is received with a load (8, 8a, 8b) of 100Ω - 10MΩ impedance.

**Patentansprüche**

1. Ein Enzymsensor mit:

einem enzymempfindlichen Abschnitt (2a,6);

einem Referenzelektrodenabschnitt (2b,7); und

einer Erfassungseinrichtung zum Aufnehmen einer Potentialdifferenz, die über dem enzymempfindlichen Abschnitt (2a,6) und dem Referenzelektrodenabschnitt (2b,7) erzeugt wird, zum Umwandeln der Potentialdifferenz in einen Strom und zum Erfassen des Stroms;

wobei die Potentialdifferenz mit einer Last (8,8a,8b) einer Impedanz von 100Ω-10MΩ aufgenommen wird.

2. Sensor nach Anspruch 1, bei dem der enzymempfindliche Abschnitt (2a,6) umfaßt:
   - eine elektrisch leitende Schicht (2a), die auf einem isolierenden Substrat (1) aufgetragen ist; und
   - eine enzymgebundene Schicht (6), die die elektrisch leitende Schicht (2a) bedeckt und ein Enzym enthält.

3. Sensor nach Anspruch 2, bei dem die elektrisch leitende Schicht (2a) eine Iridiumoxidschicht ist.

4. Sensor nach Anspruch 2 oder 3, bei dem die enzymgebundene Schicht (6) Urease trägt.

5. Sensor nach einem der Ansprüche 2 bis 4, bei dem sich der Differenzelektrodenabschnitt (2b,7) in der Nähe der enzymgebundenen Schicht (6) auf dem isolierenden Substrat (1) befindet und umfaßt:
   - eine Iridiumoxidschicht (2b) auf dem isolierenden Substrat (1)
   - eine enzymfreie Schicht (7), die die Iridiumoxidschicht (2b) bedeckt.

6. Verfahren zum Herstellen eines Enzymsensors nach einem der Ansprüche 1 bis 5, mit den Schritten
   - Ausbilden einer Mehrzahl von elektrisch leitenden Schichten (2a,2b), die auf einem isolierenden Substrat (1) voneinander getrennt sind;
   - Auftragen einer ein Enzym enthaltenden enzymgebundenen Schicht (6) auf einen Teil (2a) der elektrisch leitenden Schichten;
   - Auftragen einer enzymfreien Schicht (7) auf den verbleibenden Teil (2b) der elektrisch leitenden Schichten;
   - Anschließen der elektrisch leitenden Schicht (2a), die mit der enzymgebundenen Schicht (6) bedeckt ist, und der elektrisch leitenden Schicht (2b), die mit der enzymfreien Schicht (7) bedeckt ist, an einem Ende, das nicht in die zu untersuchende Lösung eingetaucht ist, mit einer Erfassungseinrichtung zum Aufnehmen einer Potentialdifferenz, die über den zwei elektrisch leitenden Schichten (2a,2b) erzeugt wird, zum Umwandeln der Potentialdifferenz in einen Strom und zum Erfassen des Stroms;

wobei die Potentialdifferenz mit einer Last (8,8a,8b) einer Impedanz von 100Ω-10MΩ aufgenommen wird.

**Revendications**

1. Capteur d'enzymes comprenant:

une partie sensible aux enzymes (2a, 6);

une partie formant électrode de référence (2b, 7); et

un dispositif de détection pour recevoir une différence de potentiel produite aux bornes de ladite partie sensible aux enzymes (2a, 6) et de ladite partie formant électrode de référence (2b, 7), pour convertir la différence de potentiel en un courant et pour détecter ledit courant;

dans lequel ladite différence de potentiel est reçue avec une charge (8, 8a, 8b) d'une impédance de 100 Ω à 10 MΩ.

**2.** Capteur selon la revendication 1, dans lequel ladite partie sensible aux enzymes (2a, 6) comprend:
- une couche électriquement conductrice (2a) appliquée sur un substrat isolant (1); et
- une couche à fixation d'enzyme (6) recouvrant ladite couche électriquement conductrice (2a) et contenant une enzyme.

**3.** Capteur selon la revendication 2, dans lequel ladite couche électriquement conductrice (2a) est une couche d'oxyde d'iridium.

**4.** Capteur selon la revendication 2 ou 3, dans lequel ladite couche à fixation d'enzyme (6) porte de l'uréase.

**5.** Capteur selon l'une quelconque des revendications 2 à 4, dans lequel ladite partie formant électrode de référence (2b, 7) est située à proximité de ladite couche à fixation d'enzyme (6) sur ledit substrat isolant (1) et comprend:
- une couche d'oxyde d'iridium (2b) sur ledit substrat isolant (1),
- une couche exempte d'enzyme (7) recouvrant ladite couche d'oxyde d'iridium (2b).

**6.** Procédé de fabrication d'un capteur d'enzymes selon l'une quelconque des revendications 1 à 5, comprenant les étapes de
- formation d'une multiplicité de couches électriquement conductrices (2a, 2b) séparées les unes des autres sur un substrat isolant (1);
- application d'une couche à fixation d'enzyme (6) contenant une enzyme sur une partie (2a) desdites couches électriquement conductrices;
- application d'une couche exempte d'enzyme (7) sur la partie restante (2b) desdites couches électriquement conductrices;
- connexion, à une extrémité non immergée dans la solution à examiner, de ladite couche électriquement conductrice (2a) recouverte par ladite couche à fixation d'enzyme (6) et de ladite couche électriquement conductrice (2b) recouverte par ladite couche exempte d'enzyme (7) avec un dispositif de détection pour recevoir une différence de potentiel produite aux bornes desdites deux couches électriquement conductrices (2a, 2b), pour convertir la différence de potentiel en un courant et pour détecter ledit courant;
  dans lequel ladite différence de potentiel est reçue avec une charge (8, 8a, 8b) d'une impédance de 100 Ω à 10 MΩ.

FIG. IA

FIG. IB

7

F I G. 2A

F I G. 2B

8

F I G. 2C